# EUROPEAN PATENT APPLICATION

(11) **EP 2 092 934 A1**
(43) Date of publication of application: **26.08.2009**
(21) Application number: 07831045.5
(22) Date of filing: 01.11.2007
(51) Int. Cl.: A61K 8/97, A61K 8/44, A61Q 19/00, A61Q 19/06

(54) **CERAMIDE SYNTHESIS ACCELERATORS, COSMETIC PREPARATION, SKIN PREPARATION FOR EXTERNAL USE, METHOD OF PREVENTING AGING, AND METHOD OF DIMINISHING WRINKLE**

(30) Priority: 02.11.2006 JP 2006299401
(71) Applicant: MERCIAN CORPORATION, Chuo-ku, Tokyo 104-8305 (JP)
(72) Inventor: IMAMURA, Kazuyuki, Kawasaki-shi Kanagawa 210-0818 (JP); MACHIDA, Shigeru, Kounosu-shi Saitama 365-0042 (JP)
(74) Representative: Renger, Florian
(86) International application number: PCT/JP2007/071311
(87) International publication number: WO 2008/053960

(57) **Abstract**

The present invention relates to a ceramide synthesis promoter comprising grape sap as an active ingredient, a ceramide synthesis promoter comprising grape sap and serine as active ingredients, a cosmetic preparation and an external skin preparation comprising these ceramide synthesis promoters, and method of using them. The present invention can provides a safe and effective means for improving the skin barrier function.

## Description

### CROSS-REFERENCE TO RELATED APPLICATIONS

This application claims the benefit of priority to Japanese Patent Application No. 2006-299401 filed on November 2, 2006, which is expressly incorporated herein by reference in its entirety.

### TECHNICAL FIELD

The present invention relates to a ceramide synthesis promoter capable of promoting the synthesis of ceramide, which is important to maintaining the skin barrier function, and to a cosmetic preparation and an external skin preparation capable of enhancing the skin barrier function by promoting the synthesis of ceramide.
The present invention further relates to a method of skin antiaging and a method of wrinkle relieving, using the above ceramide synthesis promoter.

### BACKGROUND TECHNIQUE

Dry skin, senile xeroderma, atopic dermatitis, and the like are skin disorders resulting from abnormal skin barrier function. In recent years, ceramides have attracted attention as moisturizing components affording relief from these skin disorders. Ceramide is an intercellular lipid that connects cells together at the surface of the skin (stratum corneum), making up 40 to 65 percent of the total lipids of the stratum corneum. When ceramide diminishes due to aging or the like, the skin becomes unable to retain moisture. Not only does the skin dry out, but the barrier function decreases, causing various skin disorders. Accordingly, ceramides have been blended into cosmetic preparations, and a large number of externally applied cosmetic preparations have been proposed (see Japanese Unexamined Patent Publication (KOKAI) Heisei No. 7-165690 (referred to as "Reference 1", hereinafter); members of the English language family thereof U.S. Patent Nos. 5,665,778 and 5,959,127; and Japanese Unexamined Patent Publication (KOKAI) Heisei Nos. 7-187987 (referred to as "Reference 2", hereinafter) and 9-241143 (referred to as "Reference 3", hereinafter); which are expressly incorporated herein by reference in their entirety).

In addition to these skin cosmetic preparations, a cosmetic preparation with good skin moisturizing properties in the form of cosmetics containing grape sap is also known (Japanese Unexamined Patent Publication (KOKAI) Heisei No. 10-45564 (referred to as "Reference 4", hereinafter), which is expressly incorporated herein by reference in its entirety).

### DISCLOSURE OF THE INVENTION

The skin cosmetic preparations described in References 1 to 3 and the like anticipate components that enhance the skin's own ability to synthesize ceramides, since ceramides are water-insoluble and of high molecular weight, thus tending not to penetrate the skin. However, the effects of these skin cosmetic preparations and the like have been inadequate, and there has been concern for safety when synthesized products have been employed. Although the cosmetic preparation containing grape sap described in Reference 4 has a certain moisturizing effect and alleviating effect on chapped skin, the market is in need of cosmetic preparations that can be expected to be more effective.

Under these circumstances, the present invention was devised with the object of providing a safe and effective means for improving the skin barrier function.

The present inventors conducted extensive research into achieving the above object, resulting in the discovery that, to a surprising degree, sap extracted from the grape vine stimulates the ceramide-synthesizing system, which is most important in maintaining the skin barrier function, thereby enhancing ceramide synthesis. The present invention was devised on this basis.

The present invention relates to a ceramide synthesis promoter comprising grape sap as an active ingredient.

Another aspect of the present invention relates to a ceramide synthesis promoter comprising grape sap and serine as active ingredients.

Another aspect of the present invention relates to a cosmetic preparation comprising the above ceramide synthesis promoter.

According to one embodiment, the above cosmetic preparation comprises equal to or greater than 1 mass percent of grape sap.

According to one embodiment, the above cosmetic preparation comprises equal to or greater than 1 mass percent of grape sap and equal to or greater than 0.1 mass percent of serine.

According to one embodiment, the above cosmetic preparation comprises 0.1 to 5 mass percent of serine.

According to one embodiment, the above cosmetic preparation is a skin antiaging cosmetic preparation.

According to one embodiment, the above cosmetic preparation is a wrinkle relieving cosmetic preparation.

Another aspect of the present invention relates to an external skin preparation comprising the above ceramide synthesis promoter.

According to one embodiment, the above external skin preparation comprises equal to or greater than 1 mass percent of grape sap.

According to one embodiment, the above external skin preparation comprises equal to or greater than 1 mass percent of grape sap and equal to or greater than 0.1 mass percent of serine.

According to one embodiment, the above external skin preparation comprises 0.1 to 5 mass percent of serine.

According to one embodiment, the above external skin preparation is used for skin antiaging.

According to one embodiment, the above external skin preparation is used for wrinkle relieving.

Another aspect of the present invention relates to a method of skin antiaging, using the above ceramide synthesis promoter.

Another aspect of the present invention relates to a method of wrinkle relieving, using the above ceramide synthesis promoter.

The ceramide synthesis promoter, and the cosmetic preparation and external skin preparation comprising the same, of the present invention are highly effective for enhancing ceramide-synthesizing enzyme system, which is most important in maintaining the skin barrier function. The ceramide synthesis promoter, and the cosmetic preparation and external skin preparation comprising the same, of the present invention are excellent in moisture-retaining property, prevent the skin from chapping, and have an improving effect for diminishing the appearance of wrinkles.

BEST MODE FOR CARRYING OUT THE INVENTION
The present invention will be described in detail below.

Grape sap is tracheary water that flows in the grape plant (family *Vitis*). The type of grape sap employed in the present invention is not specifically limited other than that it be sap of a plant of family *Vitis*; the sap of the grape, *Vitis coignetiae,* or the like may be employed. Examples are grape varieties such as: Kyoho, Koshu, Kaiji, Muscat Berry A, Delaware, Cambell Early, Pione, Neo Muscat, Niagara, Concord, Steuben, Cabernet Sauvignon, Pinot Noir, Merlot, Sémillon, Riesling, and Chardonnay. Neither the region of cultivation, method of cultivation, nor the like is limited. However, sap flowing out of the pruned branches of grape in early spring, before the budding season, is preferable. In particular, grape sap collected from a grape vine within about two weeks in early spring is especially effective and exhibits a high effect.

Grape sap can be obtained by cutting the stems of grape and collecting the sap that flows out. The method of collecting the sap is not specifically limited. For example, small containers or the like can be attached at the sites of pruning so that the sap flows into them, the sap that flows out can be collected, and these can be collected in a large storage container to which preservatives or the like have been added. The use of a device capable of collecting sap from various locations at once, such as the collection device described in Japanese Unexamined Patent Publication (KOKAI) Heisei No. 10-295207, and the liquid receiving container described in Japanese Unexamined Patent Publication (KOKAI) No. 2005-118035, for collection is preferable because such collection is highly efficient and deterioration in quality is prevented.

As needed, the collected grape sap can be mixed with preservatives or the like and employed in liquid form or as a freeze-dried product. As needed, the collected grape sap can be subjected to a heat or clarification treatment. This prevents the sap from clouding or turning brown. The heat treatment can be conducted by heating the grape sap to, for example, 60 to 100°C, preferably 80 to 90°C, for equal to or longer than 10 minutes, for example. The heat treatment can be conducted on the grape sap after collection, or conducted following processing as a product. The clarification treatment can be conducted using a known clarifying agent, such as activated carbon, bentonite, silica gel, silica sol, or PVPP (polyvinyl polypyrrolidone).

The ceramide synthesis promoter of the present invention comprises grape sap as an active ingredient, and, from the perspective of achieving an effective ceramide synthesis-promoting effect, can comprise grape sap in a proportion of equal to or greater than 1 mass percent, more preferably 3 to 100 mass percent, and further preferably 5 to 80 mass percent, based on unadjusted sap. In the present invention, grape sap itself can be employed as a ceramide synthesis promoter.

The ceramide synthesis promoter of the present invention can contain grape sap in liquid form or in the form of a freeze-dried product. The formulation is not specifically limited. They can be provided in various formulations such as lotions, emulsions, gels, creams, ointments, powders, and grains.

The ceramide synthesis promoter of the present invention can comprise an active ingredient in the form of serine in addition to grape sap. The additional incorporation of serine can enhance the ceramide synthesis-promoting effect of grape sap. The serine content is determined by taking into account the content of grape sap. Employing about 0.1 to 1-fold the mass of the grape sap (based on unadjusted sap) is suitable from the perspective of enhancing the ceramide synthesis-promoting effect of grape sap.

In the ceramide synthesis promoter of the present invention, as needed, oil-based components, surfactants, moisturizers, pigments, ultraviolet radiation-absorbing agents, antioxidants, fragrance materials, dyes, antibacterial agents, fungus-combating agents, alcohols, water and the like, that are commonly blended into pharmaceuticals, quasi-drugs, skin cosmetic preparations and the like, may be suitably blended in grape sap or a freeze-dried product thereof. To the extent that the effect of the present invention is not lost, other ceramide synthesis promoters may also be used together.

The ceramide synthesis promoter of the present invention can be employed as an orally administered drug in the form of a powder or grain. In that case, the dose can be suitably selected based on conditions such as the age and skin condition of the person to whom it is being administered. For example, the daily dose for an adult falls within a range of 100 to 1,000 mg (as a quantity of the active ingredient (based on the weight of freeze-dried product)). However, this dose can be suitably varied based on the above conditions.

The ceramide synthesis promoter of the present invention can be suitably applied as a cosmetic preparation and an external skin preparation. Furthermore, it can be employed to prevent aging of the skin by alleviating dry skin and tension, and can also be employed to relieve wrinkles.
The ceramide synthesis promoter of the present invention has a particularly good ceramide synthesis-promoting effect. Thus, in addition to conventional applications as a simple cosmetic preparation, it can be applied to a new application as an external skin preparation, that is used for alleviating skin disorders caused by abnormal skin barrier function, such as dry skin, senile xeroderma, and atopic dermatitis.

In the present invention, the use of a combination of serine and grape sap with a high ceramide synthesis-promoting effect makes it possible to provide cosmetic preparations and external skin preparations with better effects for heightening the skin moisturizing effect, alleviating chapping of the skin, and diminishing the appearance of wrinkles. Serine is sometimes contained in trace amounts (for example, about 10 to 30 ppm) as a natural component of grape sap. However, in the present invention, a quantity of serine that is substantially greater than the content of serine originally contained as a natural component of grape sap is added to intensify the ceramide synthesis-promoting effect of grape sap.

Regardless of whether or not serine is added, the quantity of grape sap that is blended in when employing the ceramide synthesis promoter of the present invention, comprising grape sap, as a cosmetic preparation or an external skin preparation is desirably equal to or greater than 1 mass percent based on unadjusted sap. The blending quantity of grape sap preferably falls within a range of 2 to 80 mass percent, more preferably 3 to 50 mass percent, based on the unadjusted sap.

The blending quantity of serine when employing the ceramide synthesis promoter of the present invention, comprising grape sap and serine, as a cosmetic preparation or an external skin preparation is desirably equal to or greater than 0.1 mass percent. The blending quantity of serine, as in the case of the ceramide synthesis promoter, is determined by taking into account the content of grape sap. The use of about 0.1 to 1-fold the mass of the grape sap (based on unadjusted sap) is suitable from the perspective of enhancing the ceramide synthesis-promoting effect of grape sap. The blending effect of serine gradually increases, so there is no clear upper limit. However, the range of 0.1 to 5 mass percent is common, and appropriately, the range of 0.3 to 3 mass percent is preferable, with the range of 0.5 to 2 mass percent being more preferable. The blending quantities of grape sap and serine are ratios per 100 mass percent of the total of all components of the cosmetic preparation or external skin preparation.

The method of applying the ceramide synthesis promoter of the present invention as an external skin preparation can be suitably selected based on the type of external preparation. Generally, when applied as an external preparation prepared as a lotion, emulsion, gel, cream, or ointment, it is desirably applied to the skin of the face or the like about once or twice daily. The ceramide synthesis promoter of the present invention can also be employed as a cosmetic preparation, and can be applied as a lotion, emulsion, beauty solution, cream, liquid foundation, powder foundation, lipstick, and the like.

### EXAMPLES

Examples of the present invention will be described below. However, the present invention is not limited thereto.

### Embodiment 1

To verify the ceramide synthesis-promoting effect of grape sap, the level of expression of the ceramide synthesis rate-limiting enzyme (serine palmitoyl transferase (SPT)) mRNA was measured with human epidermal cells. SPT is an enzyme that catalyzes the condensation reaction producing 3-ketosphinganine from L-serine and palmitoyl. This substance is then converted into ceramide. That is, the greater the quantity of SPT, the more ceramide synthesis is being promoted. Accordingly, the level of SPT was evaluated as the level of SPT mRNA expression.

The change in the level of expression of ceramide synthesis rate-limiting enzyme (SPT) mRNA was evaluated in epithelial cells. Cyclophilin was employed as a control housekeeping gene.

Normal human epithelial cells were seeded at a cell density of 5.0 x 10⁴ cells/well in six-well plates employing HuMedia KG2 medium. The cells were cultured for 4 days, and then switched to HuMeia-KG2 medium, unsupplemented with bovine hypophysis extract, containing a certain concentration of sample, in which they were cultured for 48 hours. Following washing with PBS(-), the cells were broken up with 500 µL of TRlzol^{(R)} reagent (Invitrogen). A 100 µL quantity of chloroform was added and thoroughly mixed. Centrifugation was conducted, the aqueous layer of the supernatant was collected, and 2-propanol precipitation was employed to obtain total RNA. The RNA was washed with 75 percent cold ethanol, dissolved in DEPC (diethylpyrocarbonate) water, and subjected to RT-PCR.

A reverse transcription reaction was implemented according to a protocol of a Super Script First-Strand Synthesis Kit (Invitrogen) with 1 µg of total RNA. A PCR reaction was conducted with Taq DNA Polymerase (Roche) employing the cDNA obtained by reverse transcription as template with the following primers and under the following reaction conditions.

[Table 1]

**Table 1 PCR condition of SPT mRNA expression evaluation with epithelial cells**

| Gene | Primer (5) → (3') | | T*m* (°C) | Cycle |
|---|---|---|---|---|
| SPT | Sense | 5'- GTGACCACAACCCGAATG -3' (Seq. ID No.1) | 55 | 30 |
| | Anti-sense | 5'- GATTACAGGCATCCCGTAG -3' (Seq.ID No.2) | | |
| Cyclophilin | Sense | 5'- CCGGGTGATCTTTGG -3' (Seq.ID No.3) | 60 | 25 |
| | Anti-sense | 5'- GGCGATGGCAAAGGG -3' (Seq.ID No.4) | | |

The PCR product was subjected to electrophoresis in agarose gel and then dyed with SYBR Gold^{(R)} (Invitrogen) to obtain a migration image. The brightness of the various bands of the migration image obtained was quantified with a CS Analyzer Version 2.0 (Atto) to obtain the expression levels of various genes. Correction was conducted by dividing the SPT by the expression level of the cyclophilin gene, and the levels were expressed in the form of an index (%) as percentages, with the corrected value of cells cultured with no sample (control) being 100. Each gene expression level was subjected to Student's t-test for testing significant differences, with the difference with the control being evaluated. The results are given in Table 2. A significant increase in SPT mRNA expression was observed that was dependent on sample concentration at equal to or greater than 500 µg/mL of freeze-dried product of grape sap. When 1,000 mL of grape sap was freeze-dried, 3.6 g of freeze-dried product was obtained.

[Table 2]

**Table 2 Action of sample on SPT mRNA expression (n=3)**

| Concentration of freeze-dried product of grape sap (µg/mL) | Relative intensity (SPT/cyclophilin) | |
|---|---|---|
| | Index (%) ± SD | p (t-test) ¹⁾ |
| 0 | 100.0 ± 23.6 | 1.000 |
| P.C. ²⁾ | 214.9 ± 57.0 | 0.032 * |
| 500 | 214.2 ± 66.6 | 0.049 * |
| 1000 | 267.4 ± 100.1 | 0.048 * |

| | | |
|---|---|---|
| 1) A significant difference relative to cells untreated with sample (Student's t-test) 2) Positive control (P.C.): 200 µM nicotinic-acid amide The symbol, *, indicates a significant increase in expression level. | | |

From the results shown in Table 2, grape sap is found to have a ceramide synthesis-promoting effect, and the ceramide synthesis promoter of the present invention, comprising an active ingredient in the form of grape sap, was found to be useful in promoting ceramide synthesis. Since the ceramide synthesis promoter of the present invention had a ceramide synthesis-promoting effect, it is also found that it can be applied to a new application as an external skin preparation used for relieving skin disorders due to abnormalities in the skin barrier function, such as dry skin, senile xeroderma, and atopic dermatitis, in addition to conventional applications as a simple cosmetic preparation.

### Embodiment 2

### Effect of blending in serine

### [Preparation of test sample]

A cream composition of the formula shown in Table 3 was prepared to confirm the effect of a composition comprising the ceramide synthesis promoter of the present invention. The cream composition was subjected to the organoleptic evaluation tests and fine wrinkle evaluation tests indicated below. The blending quantities of the composition shown in Table 3 are all given as percentages.

**[Table 3]**

| **Starting materials** | **Comp.Ex.1** | **Ex.1** | **Ex.2** | **Ex.3** |
|---|---|---|---|---|
| Grape sap | 0.00 | 5.00 | 5.00 | 5.00 |
| L-serine | 1.00 | 0.00 | 0.50 | 1.00 |
| Xanthan gum (2 percent aqueous solution) | 5.00 | 5.00 | 5.00 | 5.00 |
| Polyethylene glycol | 1000 | 2.50 | 2.50 | 2.50 |
| Acrylic acid - alkyl methacrylate copolymer (2 percent aqueous solution) | 15.00 | 15.00 | 15.00 | 15.00 |
| Carboxyvinyl polymer (2 percent aqueous solution) | 20.00 | 20.00 | 20.00 | 20.00 |
| Purified water | 33.50 | 29.50 | 29.00 | 28.50 |
| Dipotassium glycyrrhizinate | 0.10 | 0.10 | 0.10 | 0.10 |
| Dipropylene glycol | 3.00 | 3.00 | 3.00 | 3.00 |
| 1,3-Butylene glycol | 5.00 | 5.00 | 5.00 | 5.00 |
| Concentrated glycerin | 4.00 | 4.00 | 4.00 | 4.00 |
| Phenoxyethanol | 0.40 | 0.40 | 0.40 | 0.40 |
| Decaglyceryl monomyristate | 0.50 | 0.50 | 0.50 | 0.50 |
| Triglyceryl 2-ethylhexanoate | 1.00 | 1.00 | 1.00 | 1.00 |
| Vegetable squalane | 2.00 | 2.00 | 2.00 | 2.00 |
| Decamethyl cyclopentasiloxane | 2.00 | 2.00 | 2.00 | 2.00 |
| Methyl polysiloxane | 1.00 | 1.00 | 1.00 | 1.00 |
| Potassium hydroxide | 0.35 | 0.35 | 0.35 | 0.35 |
| Purified water | 3.65 | 3.65 | 3.65 | 3.65 |
| Total | 100.00 | 100.00 | 100.00 | 100.00 |

### [Organoleptic evaluation tests]

Sixty women aged from 30's to 50's who complained of dry skin or lack of proper skin tension were employed as test subjects. They were divided into four groups of 15 each without deviation in age. The creams of the four formula examples of given in Comparative Example 1 and Examples 1 to 3 indicated in Table 3 were given to the respective groups of test subjects, who were requested to use them twice a day, once in the morning and once at night, for 30 consecutive days. An organoleptic evaluation tests were conducted on the use sensations: a moist sensation (moisturizing property), tension sensation (resilience), and smoothness sensation (lubricating property). The evaluation criteria are given in Table 4. Furthermore, the evaluation results of the organoleptic tests are given in Table 5.

**[Table 4]**

| Use sensation | Evaluation score | Evaluation |
|---|---|---|
| Moist sensation (moisturizing property) | 5 | Very moist |
| | 4 | Moist |
| | 3 | Normal |
| | 2 | Not moist |
| | 1 | Not moist at all |
| Tension sensation (resilience) | 5 | Very tensional |
| | 4 | Tensional |
| | 3 | Normal |
| | 2 | Not tensional |
| | 1 | Not tensional at all |
| Smoothness sensation (lubricating property) | 5 | Very smooth |
| | 4 | Smooth |
| | 3 | Normal |
| | 2 | Not smooth |
| | 1 | Not smooth at all |

**[Table 5]**

| | Comp.Ex.1 | Ex.1 | Ex.2 | Ex.3 |
|---|---|---|---|---|
| Moist sensation (moisturizing property) | 2.9 | 3.9 | 4.2 | 4.4 |
| Tension sensation (resilience) | 2.8 | 3.3 | 3.9 | 4.5 |
| Smoothness sensation (lubricating property) | 2.8 | 3.1 | 3.7 | 4.1 |
| Average value | 2.8 | 3.4 | 3.9 | 4.3 |

### [Evaluation test of fine wrinkle relief]

Forty women aged from 40's to 60's who were concerned about skin wrinkles (particularly crow's feet) were employed as test subjects. They were divided into four groups of 10 each without deviation in age. The creams of the four formula examples of given in Comparative Example 1 and Examples 1 to 3 indicated in Table 3 were given to the respective groups of test subjects, who were requested to use them twice a day, once in the morning and once at night, focusing on the areas around the eyes where wrinkles tend to form, for 30 consecutive days. Crow's feet were measured by two-dimensional image analysis before and after the application, and the fine wrinkle ratios were compared. The results are given in Table 6. The two-dimensional image analysis was conducted by the method described in the following reference: Tatsuyuki Takeda et al., ed., The Utility of Cosmetics - Progress in Evaluation Techniques and Future Prospects -, Yakuji Nippo, Ltd. p. 170 (2001).

**[Table 6]**

| | Average fine wrinkle ratio (%) | | Improvement rate |
|---|---|---|---|
| | Before application | After application | |
| Comp.Ex.1 | 17 | 15 | 12% |
| Ex.1 | 18 | 12 | 33% |
| Ex.2 | 19 | 11 | 42% |
| Ex.3 | 18 | 8 | 56% |

| | | | |
|---|---|---|---|
| ⊚ Average fine wrinkle ratio: Average value of the fine wrinkle ratios of 10 individual test subjects. ⊚ Improvement rate: (fine wrinkle ratio before application - fine wrinkle ratio after application)/(fine wrinkle ratio before application) x 100 (%) | | | |

### [Test Results]

Both the results of the organoleptic evaluation test given in Table 5 and the results of the fine wrinkle evaluation test given in Table 6 indicate that the cream of the formula comprising grape sap (Example 1) exhibited a greater effect in improving dryness, tension, wrinkles, and the like than the cream having a formula comprising just serine (Comparative Example 1), and that the creams having formulas comprising grape sap and serine in combination (Examples 2 and 3) exhibited greater effects in improving dryness, tension, wrinkles, and the like than when either of these ingredients was employed alone.

The present invention is useful in the fields of cosmetic preparations, external skin preparations, and the like.

## Claims

1. A ceramide synthesis promoter comprising grape sap as an active ingredient.

2. A ceramide synthesis promoter comprising grape sap and serine as active ingredients.

3. A cosmetic preparation comprising the ceramide synthesis promoter according to claim 1 or 2.

4. The cosmetic preparation according to claim 3, comprising equal to or greater than 1 mass percent of grape sap.

5. The cosmetic preparation according to claim 3, comprising equal to or greater than 1 mass percent of grape sap and equal to or greater than 0.1 mass percent of serine.

6. The cosmetic preparation according to claim 5, comprising 0.1 to 5 mass percent of serine.

7. The cosmetic preparation according to any of claims 3 to 6, which is a skin antiaging cosmetic preparation.

8. The cosmetic preparation according to any of claims 3 to 6, which is a wrinkle relieving cosmetic preparation.

9. An external skin preparation comprising the ceramide synthesis promoter according to claim 1 or 2.

10. The external skin preparation according to claim 9, comprising equal to or greater than 1 mass percent of grape sap.

11. The external skin preparation according to claim 9, comprising equal to or greater than 1 mass percent of grape sap and equal to or greater than 0.1 mass percent of serine.

12. The external skin preparation according to claim 11, comprising 0.1 to 5 mass percent of serine.

13. The external skin preparation according to any of claims 9 to 12, which is used for skin antiaging.

14. The external skin preparation according to any of claims 9 to 12, which is used for wrinkle relieving.

15. A method of skin antiaging, using the ceramide synthesis promoter according to claim 1 or 2.

16. A method of wrinkle relieving, using the ceramide synthesis promoter according to claim 1 or 2.


SEQUENCE LISTING
<110> Mercian Corporation
<120> Reagent for promoting ceramide synthesis, and cosmetics and externa
l medicine for skin
<130> A75187H
<160> 4
<210> 1
<211> 18
<212> DNA
<213> Artificial
<400> 1
gtgaccacaa cccgaatg 18

<210> 2
<211> 19
<212> DNA
<213> Artificial
<400> 2
gattacaggc atcccgtag 19

<210> 3
<211> 15
<212> DNA
<213> Artificial
<400> 3
ccgggtgatc tttgg 15

<210> 4
<211> 15
<212> DNA
<213> Artificial
<400> 4
ggcgatggca aaggg 15